(19) Europäisches Patentamt
European Patent Office
Office européen des brevets

(11) Veröffentlichungsnummer: **0 076 378**
**B1**

(12) # EUROPÄISCHE PATENTSCHRIFT

(45) Veröffentlichungstag der Patentschrift: **02.01.85**

(51) Int. Cl.³: **C 07 C 69/72**, C 07 C 67/31

(21) Anmeldenummer: **82107751.8**

(22) Anmeldetag: **24.08.82**

(54) **Verfahren zur Herstellung von 4-Alkoxyacetessigestern.**

(30) Priorität: **01.10.81 CH 6320/81**

(43) Veröffentlichungstag der Anmeldung:
**13.04.83 Patentblatt 83/15**

(45) Bekanntmachung des Hinweises auf die Patenterteilung:
**02.01.85 Patentblatt 85/1**

(84) Benannte Vertragsstaaten:
**AT BE CH DE FR GB IT LI LU NL**

(56) Entgegenhaltungen:
**CH - A - 562 191**
**CH - A - 570 354**

**JOURNAL OF THE CHEMICAL SOCIETY, Perkin transactions I, 1979, London T. KATO et al. "Studies on Keten and its Derivatives. Part 89. Ethyl 4-Substituted Acetoacetates: Synthesis and Reaction with Diketen", Seiten 529 bis 532**

(73) Patentinhaber: **LONZA AG, Gampel/Wallis (CH)**

(72) Erfinder: **Abächerli, Claudio, Dr.Dipl.-Chem.ETH, Bäretstrasse 8, Visp (CH)**

(74) Vertreter: **Weinhold, Peter, Dr. et al, Siegfriedstrasse 8, D-8000 München 40 (DE)**

EP 0 076 378 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft ein Verfahren zur Herstellung von 4-Alkoxyacetessigestern. Es ist bekannt, 4-Ethoxyacetessigester durch Reaktion von Bromessigester und Ethoxyessigester mit Zink (J. Amer. Chem. Soc., 68, 1946, 2392) oder durch Reaktion von Ethoxyessigester mit Essigester in Gegenwart von Natrium (Chem. Abstr., 43, 1949, 2625e) herzustellen. Es ist auch bekannt, den 4-Methoxyacetessigester durch Kondensation von Methoxyacetylchlorid mit Malonsäureäthyltertiärbutylester mit nachfolgender Verseifung und Decarboxylierung herzustellen (J. Amer. Chem. Soc., 70, 1948, S. 500). Die nach diesem Verfahren erzielten Ausbeuten liegen bei 11, 21 bzw. 40%.

Versuche, 4-Ethoxyacetessigester aus 4-Chloracetessigester mit äquimolaren Mengen Na-Alkoholaten herzustellen in Alkohol, schlugen fehl. Anstelle des erwarteten 4-Ethoxyacetessigesters wurde nämlich Succinylbernsteinsäureester erhalten (Bull. Soc. Chim. France, 4. Serie, 29, 1921, S. 402—406).

Nach Schweizer Patent 562 191 ist es dann doch gelungen, 4-Alkoxyacetessigester aus den 4-Halogenessigestern mit Alkalialkoholaten herzustellen, wenn man in einem Gemisch von Alkohol und aprotischem Lösungsmittel mit hoher Dielektrizitätskonstante, vorzugsweise Dimethylsulfoxid bei Temperaturen von 15 bis 30° C arbeitet. Der Nachteil dieses Verfahrens besteht darin, daß Reaktionszeiten von 24 bis 72 Stunden benötigt werden und die Reaktion in einer großen Menge des Lösungsmittelgemisches durchgeführt werden muß.

Nach T. Kato (J. Chem. Soc., Perkin I, 529, [1979]) ist es bekannt, 4-Ethoxyacetessigester aus 4-Bromacetessigester herzustellen. Dabei wird 1 Äquivalent 4-Bromacetessigester mit 2,2 Äquivalenten Natriumethylat umgesetzt. Als Lösungsmittel dienen große Mengen Ethanol, die Ausbeuten sind mit 47% sehr bescheiden.

Der Erfindung liegt die Aufgabe zugrunde, ein Verfahren zur Herstellung der bisher schwer zugänglichen 4-Alkoxyacetessigester zu finden, das hohe Ausbeuten bei einfacher Durchführung und kurzer Reaktionszeit gestattet.

Erfindungsgemäß wird dies dadurch erreicht, daß man 4-Chlor- bzw. 4-Bromacetessigester mit Alkalialkoholat im Verhältnis von größer als 1,0 Mol Alkalialkoholat je Mol 4-Halogenacetessigester in einem aprotischen Lösungsmittel, zweckmäßig bei Temperaturen von 50 bis 100° C umsetzt.

Man arbeitet also in Abwesenheit von Alkohol. Als aprotisches Lösungsmittel wird eines mit hoher Donorzahl verwendet.

Die für die vorliegende Erfindung geeigneten Lösungsmittel können durch die Donorzahl als physikalische Konstante definiert werden. Die Donorzahl wird als physikalische Lösungsmittelkonstante definiert bei V. GUTMANN, Coordination Chemistry in Non-Aqueous Solutions, Springer-Verlag, Wien-New York, 1968 als

$$^{DN}SbCl_5 = -\Delta H_D \cdot SbCl_5$$

d. h. die Donorzahl (Donor Number) $^{DN}SbCl_5$ ist die negative Reaktionsenthalpie eines Donorlösungsmittels mit Antimonpentachlorid, gemessen in 1,2-Dichloräthan. Zweckmäßig werden Lösungsmittel, definiert durch eine relativ hohe Donorzahl, am besten über 11, angewendet.

Solche Lösungsmittel sind beispielsweise Dimethylsulfoxid, Dimethylformamid, Formamid, N-Methylformamid, N-Methylpropionamid, Dioxan, Tetrahydrofuran, vorzugsweise aber Acetonitril oder Propionitril.

Die Menge an aprotischen Lösungsmittel ist nicht kritisch; sie sollte aber zweckmäßig mindestens so groß sein, daß das Reaktionsgemisch rührbar bleibt.

Beim Arbeiten mit den genannten aprotischen Lösungsmitteln werden farblose bis schwachgelbe Rohprodukte erhalten.

Das Alkalialkoholat wird vorteilhaft in Mengen von 2,0—10 Mol, vorzugsweise 2—3 Mol, pro Mol 4-Halogenacetessigester angewendet.

Die Reaktionstemperatur liegt zweckmäßigerweise bei 50—100° C, vorzugsweise bei 60—80° C.

Als Alkalialkoholate kommen vorteilhaft die Natrium- und Kaliumsalze zur Anwendung. Als Alkoholkomponente können alle, die sich von aliphatischen Alkoholen ableiten, insbesondere solche die 1 bis 10 C-Atomen im Molekül, die geradkettig oder verzweigtkettig sein können, verwendet werden. Solche Alkohole sind beispielsweise Methanol, Äthanol, Butanol, sek. Butanol, Propanol, Isopropanol.

Von den 4-Chlor- bzw. 4-Bromacetessigestern kommen insbesondere die 4-Chlorderivate zur Anwendung. Dabei können auch solche eingesetzt werden, die in 2-Stellung einen Substituenten tragen. Somit gelingt es nach dem Verfahren der Erfindung 4-Alkoxyacetessigester der allgemeinen Formel

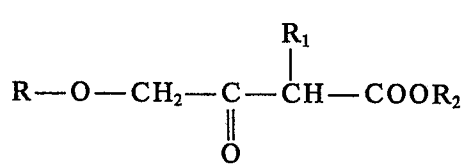

in welcher R und $R_2$ Alkylgruppen und $R_1$ = H oder Alkyl ist, herzustellen.

Die Substituenten R, $R_1$ und $R_2$ unterliegen keiner zahlenmäßigen Beschränkung der C-Atome. Beispielsweise können sie je 1 bis 10 Kohlenstoffatome aufweisen. Es können alle Alkylgruppen, ob geradkettig oder verzweigt, Anwendung finden. Auch ist es möglich, substituierte Alkylgruppen zu verwenden (z. B. Methoxy, Alkyl, z. B. $C_1-C_{10}$-Alkyl, Aryl, z. B. Phenyl). Wesentliches Merkmal der Substituenten muß sein, daß sie in stark basischem Medium nicht reagieren.

Besonders geeignet ist das Verfahren der Erfindung zur Herstellung von 4-Alkoxyacetessigestern, bei denen die 4-Alkoxygruppe und die Alkoholgruppe des Esters gleich sind. Wendet man dieses Verfahren auf die Herstellung von 4-Alkoxyacetessigestern an, bei denen die 4-Alkoxygruppe verschieden von der Alkoholgruppe des Esters ist, kann man durch Umesterung Estergemische erhalten.

Die erfindungsgemäße Reaktion benötigt nur eine kurze Reaktionszeit. Zweckmäßig wird die Reaktion während 20—40 Minuten durchgeführt. Aus dem nach der Reaktion vorliegenden Reaktionsgemisch wird der 4-Alkoxyacetessigester vorzugsweise nach folgender Methode gewonnen.

Das Reaktionsgemisch wird unter Rühren in eine im Eisbad gekühlte Vorlage aus Essigwasser, z. B. bereitet aus Eisessig und Wasser, eingeleitet. Dabei dient der Eisessig ausschließlich als Pufferkomponente um zu große pH-Schwankungen zu vermeiden. Die Menge Wasser ist zweckmäßig so einzustellen, daß keine Ausfällung von NaCl stattfindet. Andererseits soll nur soviel Wasser vorhanden sein, damit eine Phasentrennung noch stattfindet.

Gleichzeitig mit dem Zudosieren des Reaktionsgemisches wird konzentrierte Salzsäure so zudosiert, daß ein pH-Wert, zwischen 4,5 und 8, am Ende der Neutralisation von $6 \pm 1,0$ erzielt wird.

Dieses neutralisierte Gemisch wird stehen gelassen, die sich bildenden Phasen getrennt, die wäßrige Phase mit dem aprotischen Lösungsmittel, vorzugsweise Acetonitril extrahiert und die beiden organischen Phasen vereint. Aus diesen organischen Phasen wird der 4-Alkoxyacetessigester destillativ gewonnen.

## Beispiele

1. 77,4 g Natriummethylat, 97%ig wurden in 100 g Acetonitril bei Raumtemperatur suspendiert. Zu dieser gut gerührten Suspension tropfte man durch einen Tropftrichter mit Tropfenzähler innert 5—6 Minuten unter $N_2$-Atmosphäre 101,9 g 97,5%igen 4-Chloracetessigsäuremethylester zu. Die Temperatur stieg an und wurde durch Kühlen auf 68—70° C gehalten. Sobald die Wärmeentwicklung nachließ, wurde das Kühlwasser abgeschaltet und an seine Stelle mit 70° C heißem Wasser gewärmt. Dieses breiartige, senfgelbe Reaktionsgemisch wurde 24—25 Minuten bei 70° C weitergerührt und dann in eine im Eisbad gekühlte Lösung aus 6 g Eisessig und 215 g Wasser unter Rühren langsam eingeleitet. Gleichzeitig ließ man aus einer Burette 37,4%ige Salzsäure eintropfen. Dabei wurde der pH mittels einer Glaselektrode auf 4,5 bis 8 gehalten. Am Schluß der Neutralisation war der pH $6,1 \pm 0,1$. Zur Neutralisation wurden 56,4 ml Salzsäure (37,4%ig) gebraucht. Während der Neutralisation wurde die Temperatur auf 30 bis 35° C gehalten. Das Neutralisationsgemisch wurde in einen Scheidetrichter gebracht. Nach kurzem Stehen wurden die Schichten getrennt. Die wäßrige Schicht wurde einmal mit 200 ml und dann zweimal mit je 100 ml Acetonitril extrahiert. Die vereinigten organischen Phasen wurden im Rotationsverdampfer bei 30 bis 35° C und ~20 Torr bis zur Gewichtskonstanz eingedampft. Das abgedampfte Lösungsmittel wurde regeneriert und für einen nächsten Ansatz verwendet. Das Rohprodukt wurde bei 0,5 bis 1,5 Torr/90° C destilliert. Man erhielt 4-Methyloxyacetessigsäuremethylester in einer Ausbeute von 91,7%, bezogen auf den eingesetzten Chlorester. Der Gehalt des Produktes betrug 98,8%.
2. In gleicher Weise wie in Beispiel 1 beschrieben, wurden 108,6 g 4-Chloracetessigsäureethylester in 143 g Acetonitril mit 103,6 g Natriumethylat umgesetzt. Man erhielt 4-Ethoxyacetessigsäureethylester in einer Ausbeute von 90,4%, bezogen auf den eingesetzten 4-Chloracetessigsäureethylester. Der Gehalt des Produktes betrug 98,5%.
3. In gleicher Weise wie in Beispiel 1 beschrieben, wurden 101,9 g 4-Chloracetessigsäuremethylester in 180 ml Acetonitril mit 84,6 g Natriummethylat bei 60° C umgesetzt. Ausbeute 88,0%, Gehalt 98,9%.
4. Wie in Beispiel 1, allerdings bei 80° C. Ausbeute 83,9%, Gehalt 98,9%.
5. Wie in Beispiel 1, allerdings in 117 g Propionitril als Lösungsmittel. Ausbeute 90,3%, Gehalt 99,1%.
6. Wie in Beispiel 1, allerdings in 116 g Isobutyronitril als Lösungsmittel. Ausbeute 78,7%, Gehalt 97,6%.
7. In gleicher Weise wie in Beispiel 1 beschrieben, wurden 101,9 g 4-Chloracetessigsäuremethylester in 150 ml Tetrahydrofuran mit 86,4 g Natriummethylat versetzt. Ausbeute 83%, Gehalt 99,1%.
8. In gleicher Weise wie in Beispiel 1 dargestellt, wurden 130,0 g 4-Chloracetessigsäurebutylester mit 147,4 g Natriumbutylat versetzt. Ausbeute 88,9%, Gehalt 97,8%.

## Patentansprüche

1. Verfahren zur Herstellung von 4-Alkoxyacetessigsäureestern aus 4-Chlor- bzw. 4-Bromacetessigestern, dadurch gekennzeichnet, daß man 4-Chlor- bzw. 4-Bromacetessigester mit mehr als einem Äquivalent Alkalialkoholat in einem aprotischen Lösungsmittel, zweckmäßig bei Temperaturen von 50 bis 100° C, umsetzt.

2. Verfahren nach Anspruch 1, dadurch gekennzeichnet, daß man die Umsetzung in Gegenwart von Acetonitril oder Propionitril durchführt.

3. Verfahren nach Anspruch 1—2, dadurch gekennzeichnet, daß man pro Mol 4-Chlor- bzw. 4-Bromacetessigester mindestens 2,0 Mol Alkalialkoholat anwendet.

4. Verfahren nach Anspruch 1—3, dadurch gekennzeichnet, daß man bei Temperaturen von 60 bis 80° C arbeitet.

## Claims

1. Process for the production of 4-alkoxyacetoacetic acid esters from 4-chloro- and 4-bromoacetoacetic acid ester, resp., characterized in that 4-chloro- and 4-bromoacetoacetic acid ester, resp., are reacted with more than one equivalent of alkali metal alcoholate in an aprotic solvent, suitable at temperatures of from 50 to 100° C.

2. Process according to claim 1, characterized in that the reaction is carried out in the presence of acetonitrile or proprionitrile.

3. Process according to claim 1 and 2, characterized in that at least 2,0 moles of alkali metal alcoholate are employed per mol of 4-chloro- and 4-bromoacetoacetic acid ester, resp.

4. Process according to claims 1 to 3, characterized in that temperatures of from 60 to 80° C are employed.

## Revendications

1. Procédé de préparation d'esters d'acides 4-alcoxyacétylacétiques à partir d'esters d'acides 4-chloro- ou respectivement 4-bromoacétylacétiques, caractérisé en ce que l'on fait réagir de l'ester d'acide 4-chloro ou respectivement 4-bromoacétylacétique avec plus d'un équivalent d'alcoolate alcalin dans un solvant aprotique, avantageusement à des températures de 50 à 100° C.

2. Procédé selon la revendication 1, caractérisé en ce que la réaction est effectuée en présence d'acétonitrile ou de propionitrile.

3. Procédé selon la revendication 1—2, caractérisé en ce qu'on utilise par mole d'ester d'acide 4-chloro ou respectivement 4-bromoacétylacétique au moins 2,0 moles d'alcoolate alcalin.

4. Procédé selon la revendication 1—3, caractérisé en ce qu'on travaille à des températures de 60 à 80° C.